# EUROPEAN PATENT SPECIFICATION

(11) **EP 3 380 095 B1**
(45) Date of publication and mention of the grant of the patent: **05.01.2022**
(21) Application number: 16869210.1
(22) Date of filing: 23.11.2016
(51) Int. Cl.: A61K 31/352, A61K 31/192, A61K 9/00, A61P 9/10, A61K 45/06

(54) **COMPOSITIONS AND METHODS FOR TREATING ISCHEMIC STROKE**
ZUSAMMENSETZUNGEN UND VERFAHREN ZUR BEHANDLUNG VON ISCHÄMISCHEM SCHLAGANFALL
COMPOSITIONS ET MÉTHODES DE TRAITEMENT D'UN AVC ISCHÉMIQUE

(30) Priority: 23.11.2015 US 201562258882 P
(43) Date of publication of application: 03.10.2018
(73) Proprietor: The General Hospital Corporation, Boston, MA 02114 (US)
(72) Inventor: ELMALEH, David, Newton, MA 02459 (US)
(74) Representative: Wynne-Jones IP Limited
(86) International application number: PCT/US2016/063462
(87) International publication number: WO 2017/091644

(56) References cited:
- WO-A1-2018/045217
- US-A1- 2006 051 319
- US-A1- 2006 276 455
- US-A1- 2010 266 531
- US-A1- 2012 165 366
- US-A1- 2014 140 927
- US-B2- 8 617 517
- IVAN MARINKOVIC ET AL: "Evolution of Intracerebral Hemorrhage after Intravenous Tpa: Reversal of Harmful Effects with Mast Cell Stabilization", JOURNAL OF CEREBRAL BLOOD FLOW & METABOLISM, vol. 34, no. 1, 1 January 2014 (2014-01-01), pages 176-181, XP055593575, US ISSN: 0271-678X, DOI: 10.1038/jcbfm.2013.189
- CRAIG M MCKITTRICK ET AL: "Mast Cells Promote Blood Brain Barrier Breakdown and Neutrophil Infiltration in a Mouse Model of Focal Cerebral Ischemia", JOURNAL OF CEREBRAL BLOOD FLOW & METABOLISM, vol. 35, no. 4, 7 January 2015 (2015-01-07), pages 638-647, XP055593635, US ISSN: 0271-678X, DOI: 10.1038/jcbfm.2014.239
- ESTELLE ROUSSELET ET AL: "Mouse Model of Intraluminal MCAO: Cerebral Infarct Evaluation by Cresyl Violet Staining", JOURNAL OF VISUALIZED EXPERIMENTS, no. 69, 6 November 2012 (2012-11-06), XP055594257, DOI: 10.3791/4038
- PETER J KOUDSTAAL ET AL: "Secondary Stroke Prevention in Atrial Fibrillation: Indications, Risks, and Benefits", JOURNAL OF THROMBOSIS AND THROMBOLYSIS, vol. 7, no. 1, 1 January 1999 (1999-01-01) , pages 61-65, XP055594424,

## Description

### Cross Reference to Related Applications

### Field of the Invention

The invention encompasses a method of treating ischemic stroke by administering cromolyn and/or its derivatives and optionally other mast cell inhibitors to subjects in need thereof. The method of invention further encompasses administration of anti-inflammatory drugs and vascular treatment drugs in combination with the mast cell inhibitors for treatment or as an adjuvant to clinical treatment for subjects suffering from ischemic stroke. The method may include inhibiting mast cell mediated adverse effects on brain pathology after ischemic stroke including, but not limited to, post stroke neuro-inflammation, glial activation, and neuronal loss to slow or halt cognitive decline. The invention may also encompass a potentially efficacious adjuvant for treatment of post ischemic stroke in patients with cognitive impairment (PSCI- post stroke cognitive impairment).

### Background of the Invention

Stroke is the No. 5 cause of death in the US, according to the Center for Disease Control and Prevention, and a leading cause of disability in the United States. About 795,000 Americans each year suffer a new or recurrent stroke, ischemic or hemorrhagic (American Heart Association, Heart Disease and Stroke Statistics - 2015 Update, A Report From the American Heart Association. Circulation (2015)131:434-441). Approximately 610,000 of these are first events and 185,000 are recurrent stroke events.

Ischemic stroke causes damage as a result of primary and secondary insults mediated by ischemia and inflammation. Neurons in the infarcted tissue die as a result of the initial injury whereas cells in the penumbra are affected by the rapid influx of immune cells, reactive oxygen species, and toxic inflammatory mediators.

Stroke survivors often experience medical complications and long-term disabilities such as paralysis, vision problems, speech/language problems, changes in behavioral style and memory loss. A high proportion (~30%) of stroke survivors suffers from post stroke dementia including vascular, degenerative and mixed dementia. See, (Kase et al., "Intellectual Decline After Stroke The Framingham Study," Stroke (1998), 29:805-812; Lees et. al., "Test Accuracy of Cognitive Screening Tests for Diagnosis of Dementia and Multidomain Cognitive Impairment in Stroke," Stroke (2014) 45:3008-3018; Del Ser et al., "Evolution of Cognitive Impairment After Stroke and Risk Factors for Delayed Progression," Stroke (2005) 36:2670-2675; Mok et al., "Cognitive impairment and functional outcome after stroke associated with small" 2004). Incidence of dementia and mild cognitive impairment (MCI) after stroke vary (Nys et al., "Restrictions of the Mini-Mental State Examination in acute stroke," Arch Clin Neuropsychol (2005) 20:623-9; Madureira et al., "Dementia and cognitive impairment three months after stroke," Eur J Neurol (2001) 8(6):621-627; Ihle-Hansen et al., "Incidence and subtypes of MCI and dementia 1 year after first-ever stroke in patients without pre-existing cognitive impairment," Dement Geriatr Cogn Disord (2011) 32:401-407). However, cognitive impairment after stroke is attributed not only to vascular cognitive impairment (VCI) but also the pathogenesis of Alzheimer's disease (AD) (Sun et al., "Post-stroke cognitive impairment: epidemiology, mechanisms and management," Ann Transl Med (2014) 2(8): 80), contributing to approximately 1/3 dementia cases after stroke (Desmond et al., "Frequency and clinical determinants of dementia after ischemic stroke," Neurology (2000), 54:1124-1131). According to the autopsy studies, approximately 50% of dementias are attributed to both VCI and AD (Jellinger, KA., "Alzheimer disease and cerebrovascular pathology: an update," J Neural Transm (2002) 109:813-36).

The mechanisms of post-stroke cognitive impairment include neuroanatomical lesions caused by stroke (Zekry et al., "The vascular lesions in vascular and mixed dementia: the weight of functional neuroanatomy," Neurobiol Aging (2003) 24:213-219; Szabo et al., "Hippocampal lesion patterns in acute posterior cerebral artery stroke: clinical and MRI findings," Stroke (2009) 40:2042-2045), cerebral microbleeds (Greenberg, et al., "Cerebral microbleeds: a guide to detection and interpretation," Lancet Neurol (2009) 8:165-174; Park, et al., "Pathogenesis of cerebral microbleeds: In vivo imaging of amyloid and subcortical ischemic small vessel disease in 226 individuals with cognitive impairment," Ann Neurol (2013) 73:584-593) and mixed AD with stroke.

Currently, no specific treatments for VCI, including VCI associated with stroke have been approved by the US FDA (Gorelick et al., "Vascular Contributions to Cognitive Impairment and Dementia, A Statement for Healthcare Professionals From the American Heart Association/American Stroke Association," Stroke (2011) 42:00-00). Prevention of VCI is focused on detection and control of the traditional risk factors for stroke and cardiovascular disease. The efficacy of cholinesterase inhibitors (such as donepezil, rivastigamine, and galantamine) and memantine, has been studied in cognitive, global, and daily functioning in vascular dementia.

Inflammation plays an important role in the pathogenesis of ischemic stroke and other forms of ischemic brain injury. Experimentally and clinically, the brain responds to ischemic injury with an acute and prolonged inflammatory process, characterized by rapid activation of resident cells (such as microglia), production of proinflammatory mediators, and infiltration of various types of inflammatory cells (including neutrophils, different subtypes of T cells, monocyte/macrophages, and other cells) into the ischemic brain tissue (Jin, et al., "Inflammatory mechanisms in ischemic stroke: role of inflammatory cells," J Leukoc Biol (2010) 87(5): 779-789). These cellular events collaboratively contribute to ischemic brain injury (Bona, et al., "Chemokine and inflammatory cell response to hypoxia-ischemia in immature rats," Pediatr Res (1999) 45:500-509; Silverstein, et al., "Cytokines and perinatal brain injury," Neurochem Int (1997) 30:375-383; Cowell et al., "Hypoxic-ischemic injury induces macrophage inflammatory protein-lalpha expression in immature rat brain," Stroke (2002) 33:795-801).

Microglial cells, the resident macrophages of the brain, are activated rapidly in response to brain injury (Aloisi F, "Immune function of microglia," Glia (2001) 36:165-179; Nakajima, et al., "Microglia: activation and their significance in the central nervous system," J Biochem (2001) 130:169-175). It has been shown that resident microglia are activated within minutes of ischemia onset producing proinflammatory mediators, which exacerbate tissue damage (Banati et al., "Cytotoxicity of microglia," Glia, 1993, 7:111-118; Barone, et al., "Tumor necrosis factor-α: a mediator of focal ischemic brain injury," Stroke (1997) 28:1233-1244; Rothwell, et al., "The role of interleukin 1 in acute neurodegeneration and stroke: pathophysiological and therapeutic implications," J Clin Invest (1997) 100:2648-2652), but may also protect the brain against ischemic and excitetoxic injury (Mattson, et al., "Cellular signaling roles of TGFβ, TNF α and β APP in brain injury responses and Alzheimer's disease," Brain Res Brain Res Rev., (1997) 23:47-61; Raivich, et al., "Neuroglial activation repertoire in the injured brain: graded response, molecular mechanisms and cues to physiological function." Brain Res Brain Res Rev (1999) 30:77-105; Hallenbeck, JM., "The many faces of tumor necrosis factor in stroke," Nat Med (2002) 8:1363-1368). Post ischemic microglial proliferation peaks at 48-72 h after focal cerebral ischemia and may last for several weeks after initial injury (Lalancette-Hebert, et al., "Selective ablation of proliferating microglial cells exacerbates ischemic injury in the brain," J Neurosci (2007) 27:2596-2605; Denes, et al., "Proliferating resident microglia after focal cerebral ischaemia in mice," J Cereb Blood Flow Metab (2007) 27:1941-1953). In contrast to the rapid resident microglia response, blood-derived leukocytes are recruited to the brain tissue, usually with a delay of hours to a few days (Yilmaz, et al., "Role of T lymphocytes and interferon-y in ischemic stroke," Circulation (2006) 113:2105-2112; Schilling, et al., "Microglial activation precedes and predominates over macrophage infiltration in transient focal cerebral ischemia: a study in green fluorescent protein transgenic bone marrow chimeric mice," Exp Neurol (2003) 183:25-33; Tanaka, et al., "Migration of enhanced green fluorescent protein expressing bone marrow- derived microglia/macrophage into the mouse brain following permanent focal ischemia." Neuroscience (2003) 117:531-539).

Recent studies highlighted the role of brain mast cells in the pathophysiology of brain ischemia and hemorrhage (Jin, et al., "Mast cell stabilization limits hypoxic-ischemic brain damage in the immature rat," Dev Neurosci (2007) 29(4-5):373-84; Strbian, et al., "An emerging role of mast cells in cerebral ischemia and hemorrhage," Ann Med (2009) 41(6):438-50). Jin *et al.* (Jin, 2007, Jin, et al., "Mast cells are early responders after hypoxia-ischemia in immature rat brain," Stroke (2009) 40(9):3107-12) showed that mast cell recruitment and activation of mast cells preceded responses of neurons, glia, and endothelial cells by 2 to 4 hours during hypoxia ischemia in immature brain. Early mast cell activation was suggested to contribute to cerebral histamine accumulation and damage in neonatal ischemic animal model, further supporting a role for mast cells in neonatal brain injury. In addition, mast cells appear to play a role in the tPA-mediated cerebral hemorrhages after experimental ischemic stroke and to be involved in the expansion of hematoma and edema following intracerebral hemorrhage (Strbian, et al., "Cerebral mast cells regulate early ischemic brain swelling and neutrophil accumulation," J Cereb Blood Flow Metab (2006) 26:605-612; Strbian, et al., "Mast cell stabilization reduces hemorrhage formation and mortality after administration of thrombolytics in experimental ischemic stroke," Circulation (2007) 116:411-418). Mast cells stabilization was reported to reduce hemorrhagic transformation and mortality after administration of thrombolytics in experimental ischemic stroke (Strbian, 2007). Further, inhibition of this early mast cells response by cromoglycate was shown to provide long-term protection in animal models (Strbian, 2009).

Recent studies highlighted the role of brain mast cells in the pathophysiology of brain ischemia and hemorrhage (Jin, 2007, Strbian, 2009). Jin et al. (Jin, 2007, Jin, 2009) showed that mast cells recruitment and activation of mast cells preceded responses of neurons, glia, and endothelial cells by 2 to 4 hours during hypoxia ischemia in immature brain. Early mast cells activation was suggested to contribute to cerebral histamine accumulation and damage in another neonatal ischemic model, further supporting a role for mast cells in neonatal brain injury. In addition, mast cells appear to play a role in the tPA-mediated cerebral hemorrhages after experimental ischemic stroke and to be involved in the expansion of hematoma and edema following intracerebral hemorrhage (Strbian, 2006, 2007). Mast cells stabilization was reported to reduce hemorrhagic transformation and mortality after administration of thrombolytics in experimental ischemic stroke (Strbian, 2007). Further, inhibition of this early mast cells response was shown to provide long-term protection.

Mast cells were reported to contribute to brain damage in hypoxic-ischemic insults (Strbian 2006, 2007). In experimental cerebral ischemia/reperfusion, mast cells regulated permeability of the blood-brain barrier, brain edema formation, and the intensity of local neutrophil infiltration. Importantly, the mast cells-stabilizing drug cromoglycate inhibited mast cell-mediated adverse effects on brain pathology and improved survival of experimental animals (Jin, 2009).

Cromolyn, which has been approved for use since the 1970s for the treatment of asthma and allergic rhinitis, has been shown to be mast cell stabilizer, to inhibit mast cell migration and degranulation, glial activation, and neuronal death in a model of unilateral hypoxia-ischemia in neonatal rats (Jin, 2007). Recent studies in adult rats showed that intracerebral injection of cromolyn reduced the early cerebral edema and neutrophil accumulation after transient middle cerebral artery occlusion (Strbian, 2006), as well as inhibited hemorrhage formation after tPa treatment (Strbian, 2007). Cromolyn given at the end of the hypoxic period, using a model of unilateral hypoxia-ischemia in neonatal rats, limited mast cell migration/degranulation, glial activation, and neuronal loss as assessed at 48 hours (Jin, 2007). Further, long-lasting neuroprotection was demonstrated by Jin *et al* (Jin, 2009). Mast cells stabilization during the initial 24 hours significantly limited further mast cells migration into the brain and provided neuroprotection through 4 weeks of recovery (Jin, 2009).

Marinkovic et al., Journal of Cerebral Blood Flow & Metabolism, (2014), 34, 176-181 discloses a study entitled: evolution of intracerebral hemorrhage after intravenous tPA: reversal of harmful effects with mast cell stabilization.

McKittrick C. et al., Journal of Cerebral Blood Flow & Metabolism, (2015), 35, 638-647 discloses a study entitled: mast cells promote blood brain barrier breakdown and neutrophil infiltration in a mouse model of focal cerebral ischemia.

US8617517 B2 discloses cromolyn analogs useful as imaging agents for detecting atherosclerotic plaques and for treating atherosclerosis and Alzheimer's Disease.

US2006/276455 A1 discloses the use of mast cell activation- or degranulation-blocking agent in the manufacture of a medicament for the treatment of cerebrial ischemia.

### Summary of the Invention

According to a first aspect of the invention there is provided a compound as defined in the appended claims. The invention encompasses a compound for use in treating ischemic stroke in a subject in need thereof; wherein: the compound is cromolyn or a salt thereof; the compound is micronized into particles having a size of 1 to less than 10 microns and an average particle size of 5 microns or less; and the compound is administered by inhalation in an amount from 5 mg to 20 mg per dose. In one embodiment, the compound is cromolyn. In another embodiment, the compound is cromolyn sodium.

The invention also encompasses methods further comprising administering at least one additional drug. The additional drug may be a mast cell inhibitor, anti-inflammatory drug, or vascular treatment drug. In one embodiment, the additional drug is ibuprofen. In another embodiment, the compound and the additional drug may be administered either concurrently or consecutively.

One embodiment encompasses the compound for use wherein the compound is administered in an amount of about 17.1 mg per dose. Another embodiment encompasses the compound for use wherein the compound is administered twice daily.

### Brief Description of the Figures

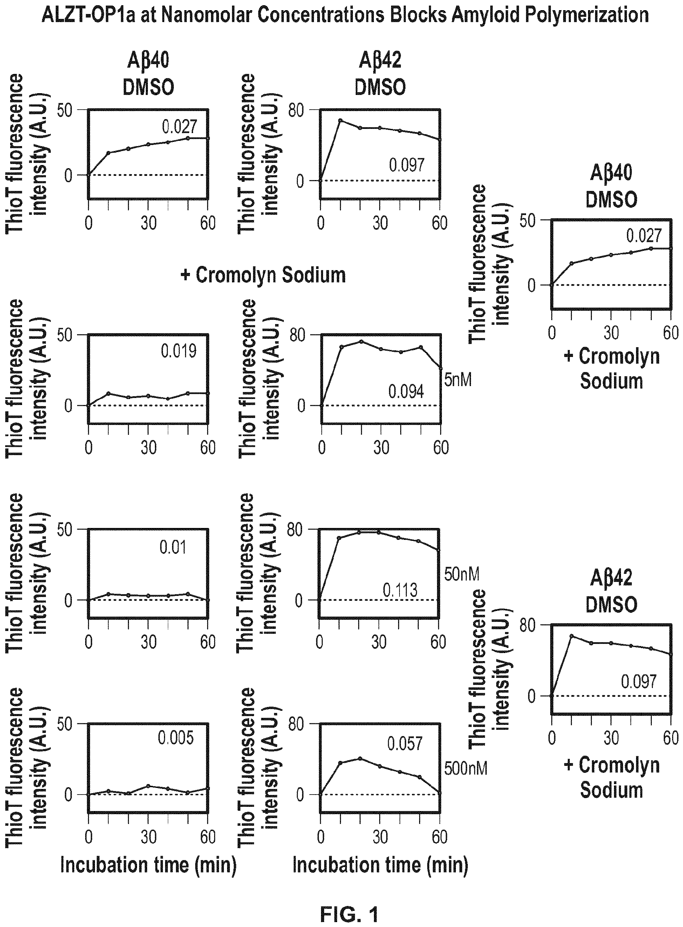

Figure 1 illustrates the inhibiting effect of cromolyn at nanomolar concentrations on Aβ (Aβ₄₀ and Aβ₄₂) aggregation. Left panel: representative curves of Thioflavin T fluorescence increase upon Aβ fibrillization after addition of DMSO (upper panel) or Cromolyn Sodium (5nM, 50nM and 500nM, lower panels). Fibrillization of synthetic Aβ₄₀ (left column) or Aβ₄₂ (right column) peptides was followed over 1 hour. The corresponding Vmax index (milli- units/minute) is indicated on each graph. Right panel: Bar graphs summarizing Aβ₄₀ (upper graph) and Aβ₄₂ (lower graph) fibrillization, which is significantly decreased in presence of Cromolyn Sodium, even though smaller effects were observed on Aβ₄₂ fibrillization process.
Figure 2 illustrates the schematic presentation of Aβ Amyloid peptide polymerization in oligomers.
Figures 3A-B illustrate views of cromolyn drug binding to Aβ-42 amyloid peptide (beta-sheet ribbon structure) from modeling structural data.
Figure 4 illustrates the results from the swimming maze memory test of ALZT-OP1 treated PS1 transgenic mice.
Figures 5A-C illustrate the reduction of the soluble monomeric Aβ, not oligomeric Aβ, in the brain of APP/PS1 mice by the acute administration of cromolyn sodium for one week.
Figure 6 illustrates the effect of cromolyn sodium microglial uptake.
Figure 7 illustrates the biodistribution of radiolabeled cromolyn in mice.

### Detailed Description of the Invention

Compounds and formulations that inhibit mast cell mediated adverse effects on brain pathology after ischemic stroke including post stroke nemo-inflammation, glial activation, and neuronal loss help slow or halt cognitive decline caused by the stroke. In an ischemic stroke, blood supply to part of the brain is decreased, leading to dysfunction of the brain tissue in that area. There are four reasons why this might happen: thrombosis, embolism, systemic hypoperfusion, or cerebral venous sinus thrombosis. A cerebral infarction is a type of ischemic stroke resulting from blockage in the blood vessels supplying blood to the brain and this loss of blood supply results is the death of tissue in that area.

This invention encompasses an adjuvant treatment in post ischemic stroke therapy. In certain embodiments, cromolyn is administered by inhalation to inhibit mast cell-mediated adverse effects on brain pathology and glial activation post ischemic stroke, and is expected to block the pathogenic cascade that leads to neurodegeneration. In persons recovering from ischemic stroke, adjuvant administration of cromolyn is posited to inhibit mast cell-mediated adverse effects on brain pathology post ischemic stroke.

The methods of the invention seek to address this effect on brain pathology and its detrimental effects by the administration of cromolyn and/or its derivatives, as well as, other mast cell inhibitors to act as mast cells stabilization for potential therapy and prevention of brain injuries in ischemic stroke and intracerebral hemorrhage.

The invention encompasses methods of treating subjects who suffered from an ischemic stroke comprising administering to the subject a therapeutically effective amount of cromolyn or a compound of Formula I. The method further encompasses the administration of at least one mast cell inhibitor, anti-inflammatory drugs, vascular treatment drugs, in combination with cromolyn or a compound of Formula I.

In particular, the invention encompasses a method of inhibiting mast cell-mediated adverse effects on brain pathology post ischemic stroke by administering a micronized form of cromolyn to a subject in need thereof to treat the effects of ischemic stroke while concurrently avoiding the toxicity effects associated with large particles of cromolyn and derivatives thereof.

Cromolyn has been shown to bind to and inhibit amyloid β (Aβ) peptide oligomerization at nanomolar concentrations in laboratory testing. The binding of cromolyn to Aβ monomers and dimers interferes with their polymerization into oligomers and higher order aggregates (Hori, et al., "FDA approved asthma therapeutic agent impacts Aβ in the brain in a transgenic model of Alzheimer's disease," J Biol Chem. (2015) 290(4): 1966-78; Elmaleh, et al., "Evaluation of F-18 Radiolabeled Cromolyn as a Potential Aβ Polymerization Inhibitor and PET Tracer," Poster at Human Amyloid Image (HAI) Conference, Miami, Florida, January 2014, see Section 2.3).

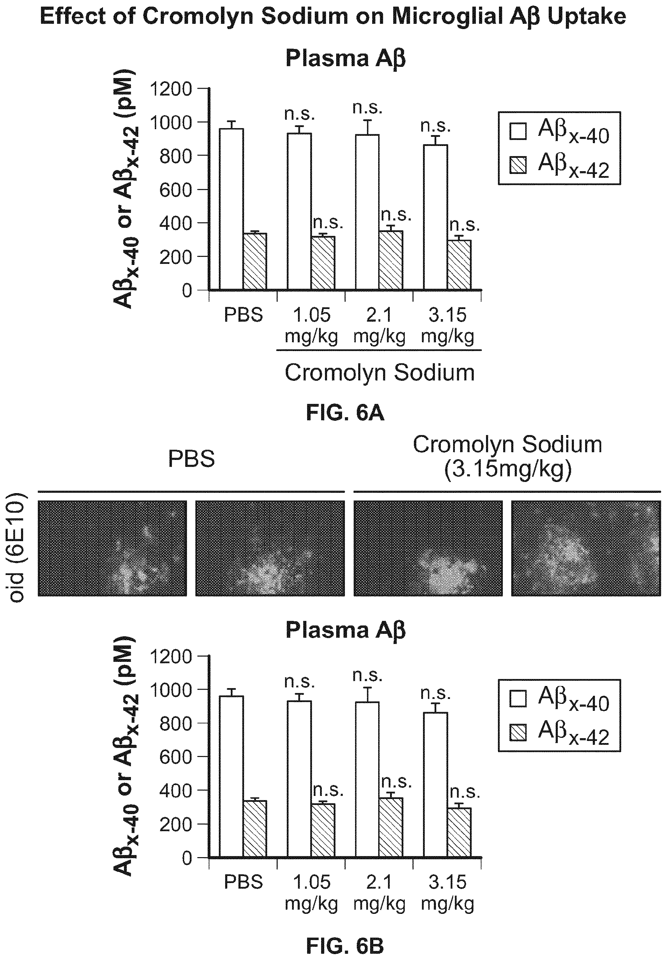

Our research indicates that cromolyn affects microglial economy. As explained below and illustrated in Figure 6, cromolyn promotes microglial Aβ clearance. The combined mechanism of action of cromolyn and mast cell inhibitors as described in Formula I or II makes these drugs candidate agents for the treatment of brain injury such as ischemic stroke and vascular dementia.

In addition to cromolyn, the compounds of the invention are illustrated by the following Formula I and Formula II: 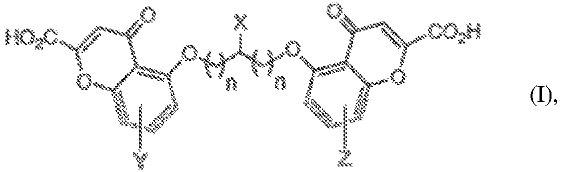 or 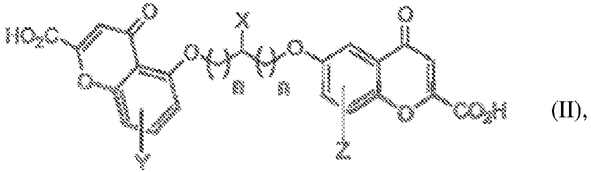
or a salt or ester of (I) or (II); wherein
X is OH, C₁-C₆ alkoxyl, ¹⁸F, or ¹⁹F;
Y and Z are independently selected from C₁-C₆ alkyl, C₁-C₆ alkoxyl, halogen, substituted or unsubstituted C₁-C₆ amine, ¹⁸F, ¹⁹F, or H; and
n is 1, 2 , or 3; and
wherein if in Formula I both Y and Z are H and n =1, then X is not OH.

Preferably, the compounds represented by formulas (I) and (II) include compounds wherein X is ¹⁸F, or ¹⁹F; Y and Z are independently selected from C₁-C₆ alkyl, C₁-C₆ alkoxyl, halogen, ¹⁸F, ¹⁹F, or H; and n is 1, 2 , or 3; and wherein if in Formula I both Y and Z are H and n =1,then X is not OH.

The compounds and methods of making the compounds used in formulations of the invention are described in U.S. Patent No. 8,617,517, Cromolyn is represented by Formula I, wherein X = OH; and Y and Z are H.

The methods of the invention include the use of cromolyn or compounds of Formula (I) and/or (II) in the treatment of subjects during post ischemic stroke therapy. Some patients may suffer from post ischemic stroke cognitive impairment (PSCI). Our studies showed that cromolyn (formula (I) where X=OH, Y, and Z are H) penetrates the blood-brain barrier in animal models, so that plasma bioavailability following cromolyn inhalation will translate to concentrations in the brain sufficient to interfere with Aβ oligomerization and accumulation.

Plasma levels of cromolyn following inhalation are reported to show high intra- and inter-subject variability, and also show that cromolyn uptake by asthmatics was lower than by healthy volunteers (Richards, et al., "Absorption and Disposition Kinetics of Cromolyn Sodium and the Influence of Inhalation Technique," J. Pharmacol. Exp. Therapeutics (1987) 241:1028-1032; Keller, et al., "Have inadequate delivery systems hampered the clinical success of inhaled disodium cromoglycate? Time for reconsideration," (2011) 8:1-17). However, the formulation of the invention prevents this deficiency by right sizing the particle of the active ingredient.

Although, the formulations of the invention may be administered using a variety of methods. Preferably, the method of administration is by inhalation. When the active ingredient is delivered by inhalation, the active ingredient is micronized to achieve an average particle size of 5 microns or less. It is important for particles to be less than 10 microns, with the majority of particles falling between 2 and 5 microns, since this is necessary for successful deposition to the secondary bronchi of the respiratory tract following inhalation. Consequently, cromolyn or the compounds of Formula (I) or (II) are micronized to a size of about 1 to less than 10 µm, preferably less than or equal to 5 µm, and more preferably less than or equal to 3 µm. For instance, the active pharmaceutical ingredient delivered by inhalation is dry powder, where the API is micronized to less than or equal to 3 µm in size. Further, the formulation of inhalation may be formulated to penetrate the lung more efficiently. Alternatively, the formulation may include an oral pill.

The active pharmaceutical ingredient may be combined with one or more mast cell affecting drugs. The two or more drugs may be delivered concurrently (e.g., as a mixture) or consecutively (e.g., as two separate delivery methods). In other embodiments, the dose is calculated to titrate the effect of the damage treated.

In addition to the compounds of the invention, the formulations of the invention further comprise at least one additional drug. These additional drugs include, but are not limited to, mast cell inhibitors, anti-inflammatory drugs, or vascular treatment drugs. The list may be by general type of drug and a second list of specific drugs that fall within the category. In one particular embodiment, the additional drug is ibuprofen.

The amount of API administered in dose will depend on a variety of conditions of the subject, such as condition of the disease, health, age, sex, weight, among others. When the formulation is formulated for inhalation, typically, the amount of cromolyn or the compounds of the invention in a single dose is about 5 to about 20 mg, preferably about 10 to 19 mg, and more preferably, the amount is about 15 to 18 mg. In one particular embodiment, that amount of cromolyn or the compounds of the invention is about 17.1 mg.

For example, a formulation may contain cromolyn powder blend prepared for use with a dry powder inhaler device. Each unit will comprise 17.1 mg of the cromolyn and pharmaceutically acceptable excipients. The formulation may be administered twice daily (34.2 mg) that is less than 50% of the cromolyn dose from the four times daily approved dose level (80 mg cromolyn total per day) currently administered for the treatment of asthma.

For daily administration, typically, the amount of cromolyn or compounds of Formula (I) or (II) would be about 5 mg to about 45 mg; preferably, the amount of the daily dose would be about 20 mg to about 38 mg, and more preferably, the amount would be about 30 gm to about 36 mg. For example, a daily dose of 34.2 mg cromolyn (17.1 mg cromolyn, inhaled twice daily, morning and evening using dry powder inhaler) would inhibit post stroke neuro-inflammation and limit mast cells migration/degranulation, glial activation, and neuronal loss and potentially slow down cognitive decline.

When administered with a second active ingredient, the cromolyn or the compounds of Formula (I) or (II) may be administered with ibuprofen. Typically, the cromolyn is administered in an amount of about 17.1 mg and ibuprofen is administered in 20 mg (such as two orally administered 10 mg doses taken consecutively). Alternatively, cromolyn of the compounds of Formula (I) or (II) is administered in 34.2 mg (such as administration of two consecutive inhaled doses of 17.1 mg) and 20 mg of ibuprofen. Preferably, the doses of cromolyn or compounds of Formula (I) or (II) are taken not more than two minutes apart.

The human pharmacokinetics data showed that cromolyn concentration in plasma reached maximum of 47.1±33.6 ng/ml (range: 14.0 - 133 ng/ ml) at 23.3± 16.9 min (range: 5-60 min) upon inhalation of 17.1 mg cromolyn dose. Cromolyn absorption by the CSF was similarly fast. Cromolyn presence in the CSF was detected 20 minutes to 2 hours following inhalation and cromolyn concentration increased for the duration of the measurement (due to limitations of the CSF collection through lumbar puncture catheter the samples could not be collected for more than 4 hours).

Cromolyn concentrations in CSF at 4 hours following 17.1 mg cromolyn inhalation was 0.24±0.08 ng/ml (range: 0.15-0.36 ng/ml), corresponding to 0.46±0.15nM. It is estimated that this level is sufficient to slow down the neuro inflammatory damage post ischemic stroke.

Cromolyn clearance from plasma was fast with mean residence time of 3.3±2.9 h (range: 0.79-12.1h), corresponding to the half-life of approximately 2.3h. At 12 hours following inhalation, cromolyn concentration in plasma would be negligible. Assuming similar clearance kinetics in CSF, chronic twice daily cromolyn inhalation regiment was chosen to maintain cromolyn concentration in plasma and CSF at sufficient levels during the day.

The chronic cromolyn or compounds of Formula (I) or (II) proposed dose taken twice daily, is estimated to slowdown or halt neuro-inflammatory damage post ischemic stroke, by titrating and controlling cytokine production, microglia activation and mast cell proliferation, without affecting potential toxicity due to chronic use of the drug.

Because cromolyn can be a hygroscopic material when the particle size distribution is suitable for inhalation (d₅₀<5 µm and d₉₀<10 µm). Therefore, the formulations of the invention may include at least one hydrophobic excipient in the powder formulation to improve product performance and stability. Addition of hydrophobic excipient offers inherent resistance of dry powder inhalation formulations to negative effect of moisture to such formulations. In one example, the hydrophobic excipient is magnesium stearate because it is commercially used in dry powder inhaler (DPI) products. Additionally, its safety profile is well studied and demonstrated for use in inhalation products. In certain embodiments, lactose monohydrate may be additionally used as diluent.

Other excipients used in the formulations of the invention include, but are not limited to, hydroxypropylmethylcellulose (HPMC). Preferably, the excipient is a clear #3 HPMC.

Exemplary formulations are shown in Table 1.

**Table 1 ALZT-OP1a (Cromolyn) Formulation**

| **Component** | **Quality Standard** | **Function** | **ALZT-OP1a Composition** | | | |
|---|---|---|---|---|---|---|
| | | | | | **Drug Product** | |
| | | | | | **%w/w** | **mg / capsule** |
| Cromolyn sodium (micronized) | USP | Active | | | 58.0 | 17.1^{a} |
| Lactose monohydrate | NF | Diluent | | | 40.0 | 12.8 |
| Magnesium stearate (micronized) | NF | Stabilizer | | | 2.0 | 0.6 |
| Hydroxypropyl methylcellulose capsule^{b} | In-house | Encapsulation | | | NA | NA |
| Total | | | | | 100% | 32 |

| | | | | | | |
|---|---|---|---|---|---|---|
| ^{a} Weight of cromolyn sodium, USP per capsules is 17.1 mg on an anhydrous basis (18.6 mg per capsule on as-is basis). ^{b} Hydroxypropyl methylcellulose capsule functions only to meter and deliver the drug product through the dry powder inhaler and is not ingested during administration. | | | | | | |

RS01 is commercially available in various versions based on airflow resistance *i.e.* 40L, 60L, 80L and 100L versions. The 40L and 60L versions are high-resistance devices and their use is limited to specific patient population range with normal respiratory function that could exclude elderly, children and patients with severe respiratory impairment.

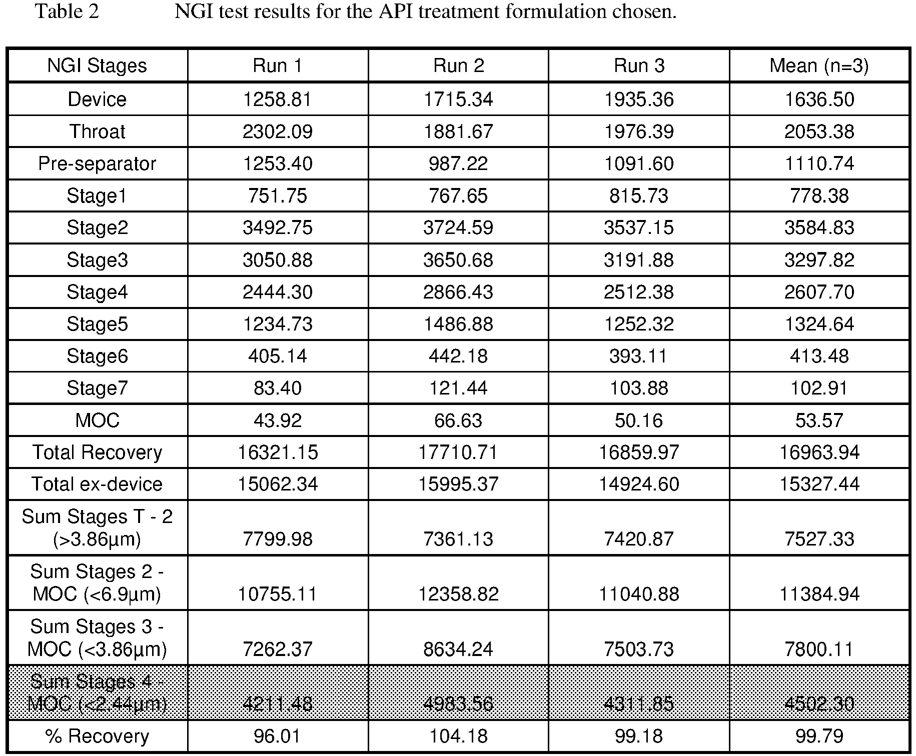

In an alternative formulation, the formulations of the invention are capsules, such as capsules for use with monodose dry powder inhaler (DPI). Gelatin or HPMC capsules are commercially used for monodose DPI products. For this purpose, HPMC capsules are used in a preferred embodiment due to their known compatibility with the RS01 DPI inhaler device. Preferably, the excipients used in the capsule formulation should be well suited for moisture sensitive drugs such as cromolyn sodium. Typically, the capsules should have low moisture content levels, typically about 4% to 6%, as compared to gelatin counterpart, which is typically 13% to 16% when measured at 50% relative humidity (RH).

One advantage of using cromolyn or the compounds of Formula (I) or (II) in the methods of the invention include lipophilicity (LogP) and polar surface area. For example, cromolyn and its fluorine analog, F analog, have LogP values of 1.39 and 2.1, respectively (Table 3). It is estimated that drugs with LogP of >3 have limited or no blood brain barrier (BBB) penetration. Other factors that affect BBB penetration are molecular size, charge and polar surface area (PSA). The PSAs of 125.43 and 127.20 for cromolyn and its F analog, respectively, are in the range of good brain penetration.

**Table 3 Calculated LogP and Polar Surface Area in Brain Penetration for Cromolyn and a Cromolyn Analog**

| Compound | Structure | MW | LogP^{∗} | PSA^{∗∗} |
|---|---|---|---|---|
| F analog | 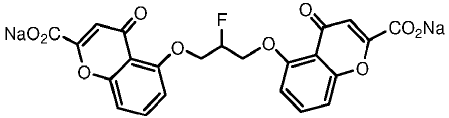 | 514.3 2 | 2.1 | 127.20 |
| Cromolyn | 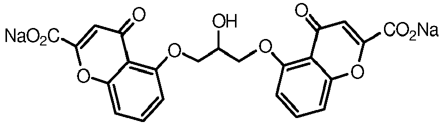 | 512.3 3 | 1.39 | 125.43 |

| | | | | |
|---|---|---|---|---|
| ^{∗} Log P was determined by ChemDraw Pro Software, Version 10 ^{∗∗} Molecular Polar Surface Area (PSA) was determined using http://www.daylight.com/meetings/emug00/Ertl/tpsa.html | | | | |

The binding of cromolyn to Aβ amyloid peptide and inhibition of its aggregation into higher order oligomers (which would get trapped in the brain) was confirmed by multiple independent assay methods. One of the most routinely used approaches to monitor Aβ polymerization is the thioflavin T binding assay (Elmaleh, 2014; Hori, 2015). When thioflavin T binds to beta-sheet rich structures, such as amyloid aggregates, the dye displays enhanced fluorescence and a characteristic red shift in its emission spectrum. Aβ peptide at 5 µM was mixed with 10 µM thioflavin T with drug at different concentrations. In the absence of drug, Aβ polymerization shows increasing thioflavin T fluorescence over 60-180 min.

Thioflavin T is only capable of binding to amyloid fibril structures, whereas ALZT-OPla (cromolyn) inhibitors bind to monomers and low order oligomer intermediates of misfolded amyloid beta peptides (5 µM Aβ without and with drug; thioflavin 10 µM; heparin 0.5 mg/ml, 200 µl assay volume) (data not shown). The addition of cromolyn at nanomolar concentration showed inhibition of Aβ aggregation, as shown in Figure 1. The left panel of Figure 1 illustrates representative curves of Thioflavin T fluorescence that increased upon Aβ fibrillization after addition of DMSO (upper panel) or cromolyn sodium (5nM, 50nM and 500nM, lower panels). Fibrillization of synthetic Aβ40 (left column) or Aβ42 (right column) peptides was followed over 1 hour. The corresponding Vmax index (milli- units/minute) is indicated on each graph. In the right panel of Figure 1, the bar graphs summarized Aβ40 (upper graph) and Aβ42 (lower graph) fibrillization, which was significantly decreased in presence of cromolyn sodium, even though smaller effects were observed on Aβ42 fibrillization process.

### Peptide Oligomer Aggregation

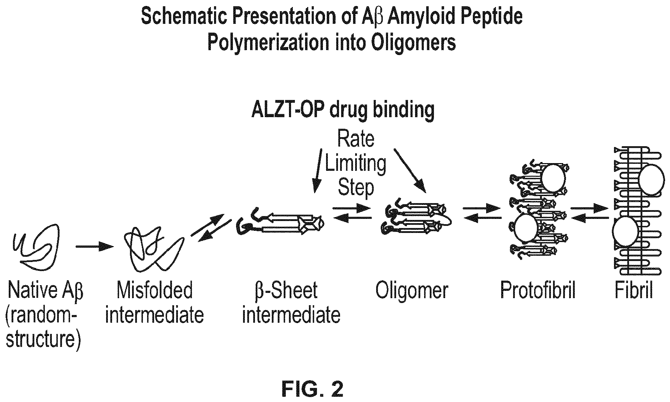

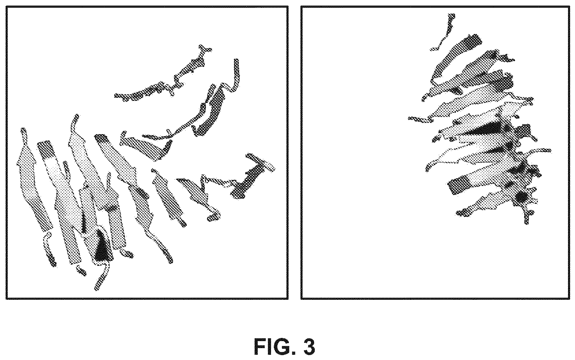

By four separate in vitro assays, cromolyn and its fluorinated derivative, effectively inhibits Aβ amyloid peptide polymerization into oligomers and higher order aggregates at nanomolar concentrations as shown in Figure 2. Figure 3 illustrates the preliminary analysis from structural data for modeling cromolyn binding to amyloid beta peptides indicates that cromolyn binding to the surface of beta strands of the amyloid peptide. The side (top panel) and Top (bottom panel) Views of Cromolyn Drug Binding to Aβ-42 Amyloid Peptide (Beta-Sheet Ribbon Structure) From Modeling of Structural Data.

### Effect on Cytokine Production

Cromolyn has been shown to be effective in a brain hypoxia-ischemia model in the mouse. Tumor necrosis alpha is a primary mediator in this model. Cromolyn-treated mice showed decrease mast cell migration, reduced brain damage/neuronal loss, decreased glial activation and decreased brain atrophy. This study showed that cromolyn targets mast cells and inhibits cytokine production, and therefore it has an additional action on treating the inflammatory response associated with the post ischemic stroke (Jin, 2009).

### Animal Models of Alzheimer's Disease

### Morris Water Navigation Test

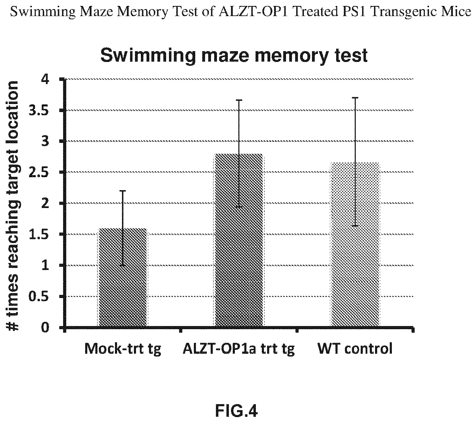

Three mice groups (five animals in each) were tested in a Morris water navigation test (unpublished data). Two groups of four-month young APP/PS1 mice, (mutant mice as animal model of AD) were tested. One APP/PS1 group was treated with cromolyn and ibuprofen combination for six months intraperitoneally twice weekly (Figure 4: group in mid panel), the second was untreated as an AD control group (Figure 4: group in left panel). The third group was an untreated wild type (WT) normal control (Figure 4: right panel). Mice were trained for 7 days to remember the location of the platform. At day 8, the platform was removed, and the times of crossing the platform area was recorded.

ALZT-OP1 treated APP/PS1 transgenic mice showed the same level of behavior as WT normal controls as compared to the decreased memory maze path recognition of the non-drug treated APP/PS1 transgenic mice (Figure 4).

### Short-term treatment of APP/PS1 mice with OLZT-OP1a

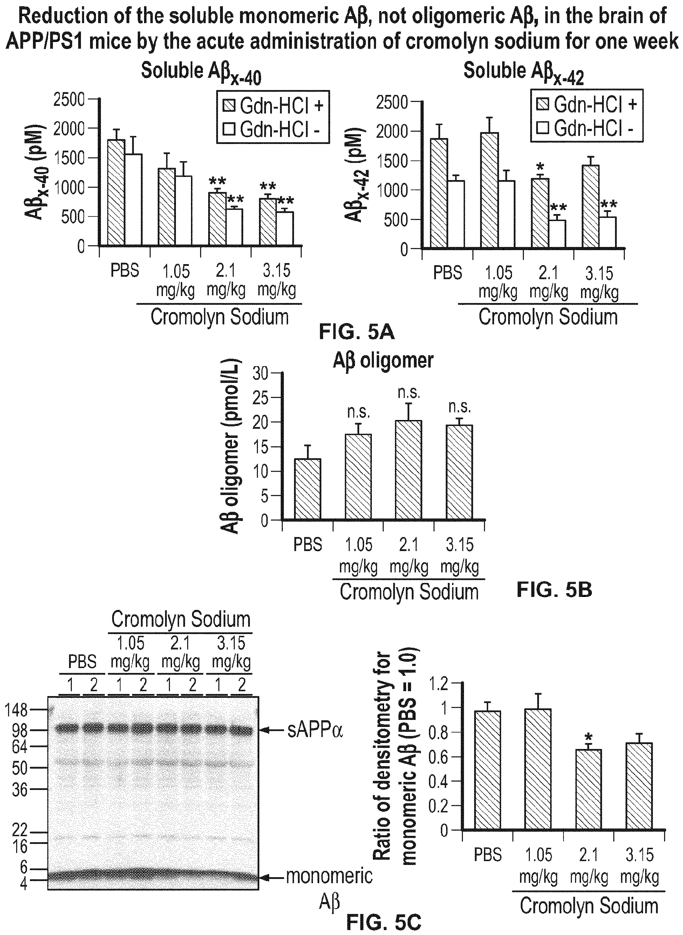

In vivo, total soluble Aβ levels are decreased by over 50% after 1 week of peripherally administered cromolyn sodium (Hori, 2015) (Figure 5). Additional microdialysis studies also show that cromolyn sodium decreases the half-life of soluble Aβ in the brain. Cromolyn sodium was injected intraperitoneally to 7.5 months old APP/PS1 mice daily for one week at three dose levels. Figure 5, panel (A) One week after daily injection, tris-buffered saline (TBS) soluble Aβx-40 (left graph) and Aβx-42 (right graph) were measured under the condition with (black bar) or without (white bar) pre-incubation with guanidine (Gdn)-HCl using Aβ ELISA. The value under the incubation with Gdn-HCl show the concentration of total TBS soluble Aβ, and the value under the no incubation with Gdn-HCl show the concentration of monomeric Aβ. Figure 5, panel (B) oligomeric Aβ in TBS soluble brain extracts were measured using 82E1/82E1 Aβ oligomer specific ELISA. Figure 5, panel (C) illustrates representative immunoblotting of TBS soluble brain extracts with anti-Aβ antibody (6E10 and 82E1) (left panel). The densitometry of monomeric Aβ was quantified (right graph). (n=3-5/group; *, P<0.05, **, P<0.01). These data suggest a clear and potent effect of a peripherally administered, FDA approved medication on Aβ economy, supporting further investigations of its potential long term efficacy in AD (Hori, 2015).

Figures 6A-B show immunostaining between Aβ and the microglial marker Iba1 in brain sections of APP/PS1 mice treated with PBS or Cromolyn sodium (3.15mg/kg). A systematic analysis of the overlap between the stains revealed that animals that received cromolyn sodium showed a higher percentage of Iba1 immunoreactivity overlapping with amyloid (upper panel), which may indicate a modest increased recruitment of microglia around plaques induced by the compound. Further, synthetic Aβ40 and Aβ42 peptides were applied to microglia culture in vitro in the presence or absence of cromolyn sodium. After 16 hours of incubation, dose- dependent decrease of Aβ40 and Aβ42 levels in presence of cromolyn sodium was observed, indicating that the impact of cromolyn sodium on Aβ aggregation mechanisms may promote Aβ clearance by microglial uptake (bottom panel). The combination of those in vivo and in vitro results may suggest that, in addition to inhibiting Aβ fibrillization, cromolyn sodium may also affect microglial activation and Aβ clearance.

In figure 6, panel A illustrates representative images of localization of amyloid deposits (6E10, green) and microglia (Iba1, red) in mice treated with cromolyn sodium (3. 15mg/kg) or PBS daily for seven days. The percentage of amyloid occupied by Iba1 positive processes was calculated for each deposit and showed an increased overlap between Aβ and Iba1 after treatment with Cromolyn Sodium (n=3 mice for PBS and n=5mice for cromolyn sodium. Between 10 to 20 plaques were evaluated for each animal). Scale bar=10 µm.

In Figure 6, panel B microglial cells were cultured and incubated with 50 nM of synthetic Aβ40 or Aβ42 and 0, 10 nM, 10 µM or 1 mM of Cromolyn Sodium for 16 hours. After the incubation, the concentrations of Aβx-40 (left panel) Aβx-42 (right panel) in media were measured using Aβ ELISA and normalized with microglia cell number and according to the PBS control condition. (n=3 experiments; *, P<0.05, **, P<0.01)

### Safety Pharmacology Studies

As formal safety pharmacology studies were not routinely conducted in the 1970s when cromolyn was approved, publicly reported data from formal animal safety pharmacology studies were not found for either of these two API components. However, extensive human data exists that indicate that the potential for these unintended effects are highly unlikely at the proposed clinical doses.

Several non-human primate studies summarized by reported studies indicated that electrocardiogram (ECG) and respiratory assessments were conducted (Beach, et al., "Cromolyn Sodium Toxicity Studies in Primates," Toxicol. Appl. Pharmacol. (1981) 57, 367-400). Results from the detailed pulmonary and cardiovascular (CV) function assessments in the inhalation studies indicated no treatment effect (Table 3).

### Biodistribution of Radiolabeled Cromolyn in Mice

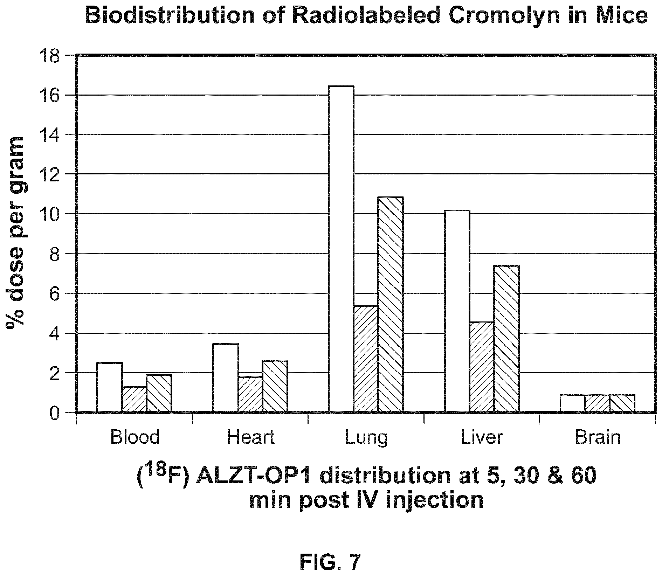

Distribution of cromolyn to different organs was investigated in mice. Radiolabeled cromolyn was injected intravenously (IV). Figure 7 shows the uptake of radiolabeled cromolyn into different organs at 5, 30, and 60 minutes after IV injection (blue, red, green bars in graph, respectively). The amount of radiolabeled cromolyn accumulated in brain tissue is 1% (dose per gram), with little or no washout over 1 hour post-injection. The highest organ accumulations were measured in lung and liver.

The effects in humans are illustrated by the pharmacokinetics (PK) of cromolyn, in plasma and CSF, following administration of a single 17.1 mg inhaled dose of cromolyn (administered with a single 10 mg dose of ibuprofen taken orally) that studied in normal, healthy volunteers between the ages of 55-75. Further, pharmacokinetics (PK) of cromolyn in plasma and CSF, following administration of 34.2 mg of cromolyn (administration of two consecutive inhaled doses of 17.1 mg taken not more than two minutes apart, administered with two 10 mg doses of ibuprofen capsules taken orally) was studied in normal, healthy volunteers between the ages of 55-75.

The results are summarized in AZTherapies' Phase I Pharamcokinetic Study Report (AZTherapies, 2015).

Upon inhalation of cromolyn single dose (17.1 mg), cromolyn plasma concentration reaches maximum of 47.1±33.6 ng/ml (range: 14.0 - 133 ng/ ml) at 23.3± 16.9 min (range: 5-60 min). Mean residence time in plasma was 3.3±2.9 h (range: 0.79-12.1h) indicating fast to moderate clearance. Cromolyn concentrations in CSF continued to increase from 2 to four 4 hr. At 4 hours following single dose inhalation was 0.24±0.08 ng/ml (range: 0.15-0.36 ng/ml).

Upon inhalation of cromolyn double dose (equal 34.2 mg), cromolyn reaches maximum plasma concentration of 95.0±45.5 ng/ml (range: 36.1-236 ng/ml) at 22.2 ±19.4 min (range: 5-60 min). Mean residence time in plasma was 2.8±1.0 h (range: 1.0-5.4 h) indicating fast to moderate clearance from the plasma.

Cromolyn concentrations in the CSF at 4 hours following double dose inhalation was 0.36± 0.17 ng/ml (range: 0.16-0.61 ng/ml). AUC (plasma) increased with the dose increase from 147.5±67.1 ng/mlxh (range: 44.7-287.0 ng/mlxh) for a single dose (17.1 mg) inhalation to 254.4±93.8 ng/mlxh (range 122.1-443.3 ng/mlxh) for a double dose (34.2 mg) inhalation. The cromolyn concentrations are estimated to effectively titrate and control cytokine production, microglia activation, and mast cell migration, without resulting in added toxicity due to chronic use of the drug.

The observed cromolyn concentration in the CSF following the 17.1 mg dose (0.46 nM), extrapolated to a doubled concentration over 8 hours, as an example translates to more than one order of magnitude (15 times) higher than the amount required to titrate the estimated daily 22-27 ng (27 ng/512 MW = 0.06 nM) of amyloidate plaque and the associated inflammatory response.

Inhaled cromolyn was transported via the deep lung to blood to brain and CSF. The flow of cromolyn from brain to CSF is slow with an increasing profile measured up to the 4hr of the lumbar puncture installed catheter. Additional CSF pharmacokinetic study should complete the uptake and washout CSF sampling curve.

Cromolyn clearance from plasma had a half-life or 1.75 ± 0.9 h (range: 0.5-3.7 h). At 12 hours following inhalation, cromolyn concentration in plasma is expected to be negligible. Assuming similar clearance kinetics in CSF, chronic, twice daily cromolyn inhalation regiment is expected to maintain cromolyn concentration in plasma and CSF at sufficient levels during the day.

The chronic, 17.1 mg proposed dose taken twice daily, is estimated to slowdown or halt neuro-inflammatory damage post-ischemic stroke, by titrating and controlling cytokine production, microglia activation, and mast cell proliferation, without affecting potential toxicity due to chronic use of the drug.

## Claims

1. A compound
for use in treating ischemic stroke in a subject in need thereof, wherein:
the compound is cromolyn or a salt thereof;
the compound is micronized into particles having a size of 1 to less than 10 microns and an average particle size of 5 microns or less; and
the compound is administered by inhalation in an amount from 5 mg to 20 mg per dose.

2. The compound for use according to claim 1, wherein the subject is further administered at least one additional drug.

3. The compound for use according to claim 2, wherein the additional drug is ibuprofen.

4. The compound for use according to claim 2, wherein the compound and the additional drug are administered either concurrently or consecutively.

5. The compound for use according to claim 1, wherein the compound is cromolyn.

6. The compound for use according to claim 1, wherein the compound is cromolyn sodium.

7. The compound for use according to claim 1, wherein the compound is administered in an amount of 17.1 mg per dose.

8. The compound for use according to claim 1, wherein the compound is administered twice daily.

## Patentansprüche

1. Verbindung
zur Verwendung bei einem Behandeln eines ischämischen Schlaganfalls bei einem Subjekt, das dies benötigt, wobei:
die Verbindung Cromolyn oder ein Salz davon ist;
die Verbindung zu Teilchen mikronisiert wird, die eine Größe von 1 bis weniger als 10 Mikrometer und eine durchschnittliche Teilchengröße von 5 Mikrometer oder weniger aufweisen; und
die Verbindung durch Inhalation in einer Menge von 5 mg bis 20 mg pro Dosis verabreicht wird.

2. Verbindung zur Verwendung nach Anspruch 1, wobei dem Subjekt ferner wenigstens ein zusätzliches Arzneimittel verabreicht wird.

3. Verbindung zur Verwendung nach Anspruch 2, wobei das zusätzliche Arzneimittel Ibuprofen ist.

4. Verbindung zur Verwendung nach Anspruch 2, wobei die Verbindung und das zusätzliche Arzneimittel entweder gleichzeitig oder nacheinander verabreicht werden.

5. Verbindung zur Verwendung nach Anspruch 1, wobei die Verbindung Cromolyn ist.

6. Verbindung zur Verwendung nach Anspruch 1, wobei die Verbindung Cromolyn-Natrium ist.

7. Verbindung zur Verwendung nach Anspruch 1, wobei die Verbindung in einer Menge von 17,1 mg pro Dosis verabreicht wird.

8. Verbindung zur Verwendung nach Anspruch 1, wobei die Verbindung zweimal täglich verabreicht wird.

## Revendications

1. Composé
destiné à une utilisation pour traiter un accident vasculaire cérébral ischémique chez un sujet en ayant besoin, dans lequel :
le composé est l'acide cromoglycique ou un sel de celui-ci ;
le composé est micronisé en particules ayant une taille de 1 à moins de 10 microns et une taille de particule moyenne de 5 microns ou moins ; et
le composé est administré par inhalation en une quantité allant de 5 mg à 20 mg par dose.

2. Composé destiné à une utilisation selon la revendication 1, dans lequel le sujet reçoit en outre au moins un médicament supplémentaire.

3. Composé destiné à une utilisation selon la revendication 2, dans lequel le médicament supplémentaire est l'ibuprofène.

4. Composé destiné à une utilisation selon la revendication 2, dans lequel le composé et le médicament supplémentaire sont administrés soit simultanément, soit consécutivement.

5. Composé destiné à une utilisation selon la revendication 1, dans lequel le composé est l'acide cromoglycique.

6. Composé destiné à une utilisation selon la revendication 1, dans lequel le composé est le cromoglycate de sodium.

7. Composé destiné à une utilisation selon la revendication 1, dans lequel le composé est administré en une quantité de 17,1 mg par dose.

8. Composé destiné à une utilisation selon la revendication 1, dans lequel le composé est administré deux fois par jour.
